# EUROPEAN PATENT SPECIFICATION

(11) **EP 1 306 084 B1**
(45) Date of publication and mention of the grant of the patent: **21.08.2013**
(21) Application number: 03075182.0
(22) Date of filing: 31.05.1999
(51) Int. Cl.: A61K 31/17, A01N 47/34, A61P 33/14

(54) **Compositions for the control of parasitic infestations in fish**
Zusammensetzungen zur Bekämpfung von parasitischen Befallen in Fischen
Compositions pour la lutte contre les infections parasitaires des poissons

(30) Priority: 09.06.1998 NO 982650
(43) Date of publication of application: 02.05.2003
(62) Divisional of application: 99921041.2
(73) Proprietor: Pharmaq AS, 7863 Overhalla (NO)
(72) Inventor: Alexander, Svein, 4024 Stavanger (NO); Evensen, Oystein, 0198 Oslo (NO); Syvertsen, Christian, 1900 Fetsund (NO); Martinsen, Bernt, 1386 Asker (NO)
(74) Representative: Plougmann & Vingtoft A/S

(56) References cited:
- WO-A-96/11707
- WO-A-96/41536
- WO-A-97/40826
- WO-A-97/45017
- WO-A-97/46204
- WO-A-99/44425
- FR-A- 2 720 898

## Description

### FIELD OF INVENTION

The present invention pertains in its broadest aspect to the field of controlling diseases in fish and in particular there are provided means for controlling parasitic infestations in fish such as farmed fish and more specifically, novel compositions for treating and/or preventing infestations with sealice and other crustacean fish parasites are provided, including injectable compositions for such treatment and/or prevention.

### TECHNICAL BACKGROUND AND PRIOR ART

Parasitic infestations constitute considerable problems in the fish farming industry as well as in wild fish. This applies especially to farmed fish in fresh water and seawater. Damages due to parasitic infestations result in considerable losses and increased workloads for the fish farmers. Infestation with sea lice (*Lepeophtheirus salmonis* and *Caligus elongatus*) is considered to be one of the most important disease problems in the farming of salmonids, especially in Atlantic salmon (*Salmo Salar*) and rainbow trout (*Oncorhynchus mykiss*). In addition to the costs that are associated with treatment, lower classification ratings of slaughtered fish and reduced growth rate due to reduced feed intake contribute to the economic losses for the fish farmer.

In addition to the damages caused in farmed fish, recent research has shown that sea lice could be the most important single cause leading to weakening of several wild salmon stocks. The emigration of salmon smolt from river systems is often coincidental with rising seawater temperatures in the fjord and coastal areas, which leads to a massive attack of copepodites (sea lice larvae) on the smolt. An attack of 40 or more sea lice on salmon smolt is fatal to fish weighing less than 25 grams, and in several regions in Norway premature return of post-smolt sea trout has been observed. The decline in the stock of salmonids that have been documented in several Norwegian salmon rivers over the past few years, could be related to the fact that the amount of sea lice in connection with fish farming has increased.

Up till now, the most common treatment of fish parasites involves bathing or immersing the fish in a treatment solution comprising an antiparasitically active compound. This includes both skin and gill parasites. Bathing in formalin is a widespread treatment against many parasites, especially in fresh water, while bathing in organophosphates (metrifonate, dichlorvos, azamethiphos), pyrethroids (pyrethrum, cypermethrin, deltamethrin) or hydrogen peroxide are the most common bath treatments against e.g. sea lice (*Lepeophtheirus salmonis, Caligus elongatus*). These substances act directly on the parasites via the water, and a possible absorption of the active substance into the fish itself is unimportant for the effect of the active substances on the parasite.

Substances that are effective against parasitic infestations for oral administration have also been tested in fish. Substances such as chitin synthesis inhibitors, diflubenzuron and teflubenzuron, and ivermectin are examples of substances, which, if administered orally, can be effective against parasitic diseases in fish. In addition to the substances mentioned above, wrasse (*Labridae*) has been used extensively to keep sea lice infestations under control.

Substances for bath treatment of parasitically infestated fish, such as sea lice infestations, must be mixed into the water where the fish normally swims. Some of the substances that are used are water-soluble (azamethiphos), while others are not (cypermethrin, deltamethrin) and therefore, the latter group of substances remain as a suspension or emulsion in water. For fish that are kept in tanks, the bath treatment can be carried out by adding the substance formulations directly into the tank where the fish are kept. For treatment of fish that are kept in sea cages, the bottom of the cage is raised manually to reduce the treatment volume. A tarpaulin is placed around the cage so that the fish are completely enclosed, whereupon the treatment substance is added to the cage. The fish swim in the solution for 20-60 minutes, depending on the treatment substance. Oxygen must be supplied to the cage in which the fish are treated. Treatment with a bottomless tarpaulin, the so-called skirt treatment, is also used to a large extent. Since the treatment volume in such cases can not be defined exactly, a larger amount of active substance will have to be used in this route of administration than is the case when a closed tarpaulin is used. It is also becoming more common to carry out bath treatments in well boats.

Organophosphates and hydrogen peroxide are only effective against the pre-adult and adult stages of sea lice (the last 3 stages of the total of 8 stages which exist on the skin of salmonids), while pyrethroids and ivermectin also have a more or less well-defined effect against the other 5 stages. None of these substances protect against new infestations after the treatment has been completed.

Hydrogen peroxide is corrosive, and must therefore be handled with great care. Transport of hydrogen peroxide requires certain precautionary measures, as it is defined as hazardous goods in great quantities. The organophosphates are toxic to humans and must therefore be treated with caution. The organophosphates could be absorbed through the skin and lead to poisoning. The therapeutic margin for organophosphates and hydrogen peroxide is small. Fish mortality has been reported on several occasions, due to overdosing of such drugs.

Pyrethroids have, in addition to their effect against pre-adult and adult lice, also an effect against the attached stages. They are not acutely toxic to the user, but is a drug group that is toxic to fish, especially small fish. Possible overdosing and increased mortality is thus possible.

All drug groups mentioned above are administered via bath treatments. This is labourintensive and might also be a stress factor to the fish.

In addition to bath treatments, oral treatments for parasite control in fish have been developed. Two chitin synthesis inhibitors, diflubenzuron and teflubenzuron, have been documented for use against sea lice in salmon. The macrocyclical lactone, ivermectin, is also being used against parasites in salmonids in Chile, Ireland and Scotland.

Diflubenzuron and teflubenzuron, which belong to the same substance group as hexaflumuron, act by inhibiting the production of chitin which is an important part of the cuticle of insects and crustaceans. At each moulting or ectdysis, a new synthesis of chitin is required for development. If this synthesis is inhibited, the development of the insect or crustacean will be halted, and the animal under development will die. In principle, chitin synthesis inhibitors will be effective against all organisms containing chitin. Fish and mammals do not contain chitin and will therefore not be affected by substances within this drug group. This is reflected in very low toxicity for fish and mammals, including humans. Diflubenzuron and teflubenzuron are administered to the fish via the feed, absorbed and distributed to skin and mucus, where the concentration will be high enough to inhibit the development of the parasite. The disadvantage of diflubenzuron and teflubenzuron is that these substances have no effect against adult stage of parasites which do not actively synthesise chitin. Neither do they have any effect beyond the period of treatment, as attack by new parasites may occur within days of completing the treatment. This is due to the fact that the substances are eliminated relatively fast so that the concentration of the substance ends up below therapeutical level in skin and mucus. The treatment must therefore be repeated if there is a continuous risk of parasite infection from the surroundings which, under normal circumstances, is often the case.

Ivermectin impairs the transfer of neural impulses in insects and crustaceans. This leads to paralysis and death. Mammals and fish are also affected by ivermectin. However, the same transfer mechanisms that are affected in insects, are only found in the brain of fish and mammals. Mammals have an advanced blood-brain barrier which prevents toxic effect from lower concentrations. The blood-brain barrier in fish is less developed and this causes a substantial transition to the brain of ivermectin in treated fish, and toxic symptoms are found at relatively low concentrations. Ivermectin, however, is effective for treatment of parasites in fish, provided that precautions with the dosing are taken. Toxic effects and mortality may rise if the fish are overdosed. Ivermectin is often dosed 1 or 2 times a week to control sea lice infestation in salmonids. This means that the substance must be added on a continuous basis to maintain therapeutic effect and keep the fish reasonably free of lice. Ivermectin is eliminated at a slow rate which means that the effect of the treatment is maintained for 2 to 3 weeks after the treatment has been completed. This also means, however, that the treatment involves a long withdrawal period before the fish may be slaughtered and consumed. Ivermectin has not been documented and approved for use on fish in any country. Sufficient documentation about withdrawal periods, toxicity to fish and to the marine environment is therefore not available.

As it appears from the above summary of the state-of-the-art, there is an industrial need for improved means of controlling parasitic infestations in fish, which are antiparasitically effective and non-toxic to the fish and can be administered in an industrialty convenient manner and which protect the fish for an extended period of time after administration. Such improvement that are provided by the invention is the treatment of fish to cure or prevent parasitic infestations by administering hexaflumuron and the administration of antiparasitically active substances by injection. Such an administration route has not been used previused previously, as it has hitherto been considered labour intensive as well as stressful for the fish and therefore, unsuitable.

WO 97/40826 and WO 99/44425 both disclose compositions comprising lufenuron in injectable form.

FR2720898 A1 describes the use of lufenuron in the management of pests, in particular Thripidae and Aculus. Various formulations of lufenuron are provided, but in practice the effect of lufenuron is investigated only on paprika plants infested by *Franklinella occidentalisi.* All specific embodiments relate to management of insect infestations within cultivation of plants. It is also suggested that lufenuron may be used in protection of domestic animals against the afore-mentioned pests; i.e. species of Thripidae and Aculus.

WO 96/41536 discloses teflubenzuron for the treatment of parasitic infestations in fish. Administration by injection is disclosed.

WO 97/45017 confirms that lufenuron and teflubenzuron fall within the same class of substituted benzoyl ureas and that they are chitin synthesis inhibitors.

WO 97/46204 provides compositions of macrolide avermectin and milbemycinendecticides with an immunizing agent and stable injectable compositions for use in warm-blooded animals.

Finally, WO 96/11707 provides the use of antigens for treatment of parasitic infestations in fish.

### SUMMARY OF THE INVENTION

Accordingly, the invention pertains in a first aspect to lufenuron for use in controlling parasitic infestations in fish, wherein said lufenuron is administered by injection.

In a further aspects the invention relates to use of lufenuron in the manufacture of a composition for controlling parasitic infestations in fish, wherein said composition is administerd by injection.

In one useful embodiment, the injectable composition contains, as a further active substance, an antigen conferring upon administration to the fish active immunological protection against viral and/or bacterial infections.

In the context of the invention, fish in which parasitic infestations are to be controlled include salmonid species such as Atlantic salmon (*Sa*/*mo salar*), rainbow trout (*Oncorhynchus mykiss*) and sea trout (*Sa*/*mo trutta*), and sea bass (*Dicentrarchus labrax*).

### DETAILED DISCLOSURE OF INVENTION

A major objective of the present invention has been to find substances and compositions for the treatment and/or prevention of infestations in fish with parasites, especially sea lice, which do not have the disadvantages of other known substances and which also protect treated fish against infections for some time after the treatment has been completed.

The invention also concerns the use of lufenuron for the manufacture of compositions for injection into fish which are useful to treat and protect against parasites, especially sea lice. Particularly interesting is the use of lufenuron in mixtures with vaccine components, for the manufacture of a composition that gives active immunological protection against bacterial and viral diseases as well as conferring prophylactic protection against parasites, especially sea lice. Combining vaccine and prophylactic treatment in one product results in protection against bacterial, viral and/or parasitic diseases. A combined product like this will neither cause additional stress to the fish nor increased workload for the fish farmer, as the use of injection vaccines against bacterial and viral diseases is already well established in the fish farming industry.

Injectable compositions according to the invention can be formulated as a solution, suspension or emulsion of lufenuron, with or without vaccine components.

The use of injectable compositions to treat and protect fish against parasitic infestations will, to a large extent, eliminate the environmental problems of today, which are associated with the use of chemical substances to control parasite problems in fish. The use of injectable compositions may cause spills to the environment in the form of secretions of the active substance from injected fish. However, this kind of spill is very limited compared to the spills from feed, faeces from oral treatment and spills directly from bath solutions after bath treatment.

As it is mentioned above, it is an important objective of the invention to provide lufenuron for use in controlling parasitic infestations in fish and the use of lufenuron in the manufacture of a composition for controlling infestations in fish with parasites such as parasitic crustacean species, wherein lufenuron is administered to the fish by injection.

This is e.g. useful in the control of infestations with sea lice species including *Lepeophtheirus salmonis* and *Caligus elongatus* and isopod species including as an example *Anilocra physodes.*

The invention will now be explained in further details in the following examples that demonstrate the effect of various formulations for injection in order to obtain prophylactic protection against parasites in fish, and in the following drawings, where:

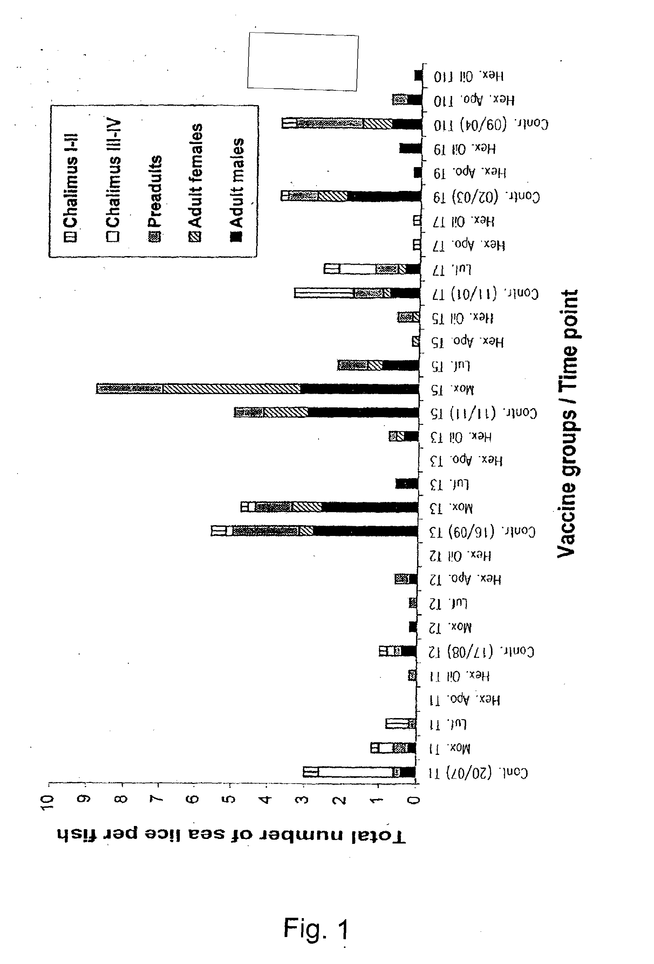

Figure 1 summarises the trial of Example 1 where salmon smolts were injected with 9 different active substances on 08.07.98. Only results for Moxidectin (Mox.), Lufenuron (Luf.) and hexaflumuron in Apoject 1-Fural (Hex.Apo) and in animal oil (Hex.oil) are shown

### EXAMPLE 1

In this trial 9 different active substances were tested for protective effect against sea lice. 1,200 salmon smolt were, prior the start of the trial, vaccinated with a commercial vaccine, ALPHA JECT 5100. This vaccine does not contain active substances conferring protection against sea lice.

One week after sea water transfer the salmon was divided into 12 groups and injected with different formulations of the 9 different active substances. In parallel with the 12 groups, a 13th group comprising 50 fish of the same origin and size was injected with a saline (PBS) solution without active substance. This group served as negative control/reference group in respect of sea lice infestations. The groups were marked by fin cutting and distributed randomly into 3 cages.

The active substances that were used as injection were hexaflumuron, buprofezin, diflubenzuron, fluazuron, lufenuron, ivermectin, doramectin, moxidectin and milbemycin oxime, respectively. All the fish were injected with 0.2 ml of the various injection formulations. Hexaflumuron, buprofezin, fluazuron, lufenuron and diflubenzuron were administered at a dosage of about 50 mg/kg whereas ivermectin, doramectin and moxidectin were administered at a dosage of 0.2 mg/kg (recommended dosage for mammals). Milbemycin oxime was administered at a dosage of 0.5 mg/kg (recommended dosage for mammals). All of the substances were administered as a formulation prepared on the basis of a vaccine against furunculosis (Apoject 1-Fural) comprising inactivated *monicida* subsp. *salmonicida.* Additionally, hexaflumuron and ivermectin were administered in a formulation prepared on the basis of an animal oil (without bacterial component) and hexaflumuron was further administered in an aqueous suspension (PBS). In total, the trial included 13 different groups including 1 control group, 3 groups injected with different formulations of hexaflumuron (Apoject 1-Fural, animal oil, PBS), 2 groups injected with different formulations of ivermectin (Apoject 1-Fural, animal oil) and the remaining substances were administered in a formulation based on Apoject 1-Fural. No adverse toxic effects were observed in any of the groups injected.

Sea lice on the fish were recorded about every third to fourth week. Figure 8 shows the average number of lice in some groups throughout the trial. At T2, i.e. 40 days after injection, only the groups injected with hexaflumuron, lufenuron or moxidectin showed protection against sea lice. At T3, i.e. 70 days after injection, only fish that had been injected with hexaflumuron formulated in Apoject 1-Fural or animal oil, and fish injected with lufenuron (Apoject 1-Fural) were protected against sea lice. On average, the control group had 5.6 lice at T3, whereas it for hexaflumuron (Apoject), hexaflumuron (animal oil) and lufenuron (Apoject) was 0, 0.8 and 0.6 lice on average, respectively at the same point in time.

Fish injected with lufenuron showed good protection against lice until T5, i.e. 128 days after injection. Hexaflumuron formulated in Apoject 1-Fural or in animal oil conferred significant protection against sea lice until and including T10. i.e. 9 months after injection.

Tissue samples (muscle/skin) for chemical analysis were collected from five fish injected with the various hexaflumuron formulations at each sample point in time throughout the trial. Fish injected with hexaflumuron in an aqueous suspension (PBS) showed a very rapid depletion of active substance and at T3, the hexaflumuron concentrations were below 0.02 µg per g. These low concentrations correlate well with the parallel recordings of sea lice which showed that there was no protection against sea lice at T3. At sea water transfer, the muscle/skin concentrations in the two other hexaflumuron groups (Apoject 1-Fural, animal oil) varied between 1 and 2.5 µg hexaflumuron/g tissue. Further analyses showed a slow depletion of hexaflumuron, i.e. 0.26 and 0.27 µg/g tissue, respectively in the two groups at T8 (218 days after injection). At T8, the number of sea lice was still significantly lower in the two hexaflumuron injected groups (Apoject 1-Fural, animal oil) as compared to the control group.

In this trial, a prophylactic effect against sea lice for up till 10 months has been demonstrated in two groups of fish injected with hexaflumuron. It has also been demonstrated that injection with other active substances is capable of conferring good protection for an extended period of time following injection. In this trial this was shown for lufenuron and moxidectin.

## Claims

1. Lufenuron for use in a method of controlling parasitic infestations in fish, wherein said lufenuron is administered by injection.

2. Use of lufenuron in the manufacture of a composition for controlling parasitic infestations in fish, wherein said composition is administered by injection.

3. Use according to claim 2, wherein the fish to which the composition is administered, is farmed fish.

4. Use according to claim 3, wherein the fish is selected from the group consisting of salmonid species including Atlantic salmon (*Salmo salar*), rainbow trout (*Oncorhynchus mykiss*), sea trout (*Salmo trutta*), and sea bass (*Dicentrarchus labrax*).

5. Use according to any of claims 2-4, wherein the composition protects the fish against infestation with a parasitic crustacean species.

6. Use according to claim 5, wherein the crustacean species belongs to sea lice species including *Lepeophtheirus salmonis* and *Caligus elongatus.*

7. Use according to claim 6, wherein the crustacean species is an isopod species including *Anilocra physodes.*

8. Use according to any of claims 5-7, wherein the composition protects against parasitic infestation for a period of at least 12 weeks after administration of the composition.

9. Use according to any of the preceeding claims, wherein said composition is for simultaneous control of parasitic infestations and infectious diseases in fish, and the composition further comprises an antigen conferring, upon administration to the fish, immunological protection against at least one viral or bacterial species.

10. Use according to any of claims 2-9, wherein said composition is formulated as a solution, suspension or emulsion of lufenuron.

11. Use according to any of claims 2-10, wherein said composition is formulated as an emulsion of lufenuron.

12. Use according to any of claims 2-10, wherein said composition is formulated as an emulsion of lufenuron with vaccine components.

## Patentansprüche

1. Lufenuron zur Verwendung in eine Verfahren für Bekämpfung von parasitärem Befall bei Fischen, wobei Lufenuron durch Injektion verabreicht wird.

2. Verwendung von Lufenuron in der Herstellung einer Zusammensetzung zur Bekämpfung von parasitärem Befall bei Fischen, wobei die Zusammensetzung durch Injektion verabreicht wird.

3. Verwendung nach Anspruch 2, wobei die Fische, denen die Zusammensetzung verabreicht wird, gezüchtete Fische sind.

4. Verwendung nach Anspruch 3, wobei der Fisch ausgewählt ist aus der Gruppe, bestehend aus einer Spezies der Salmoniden einschließlich Atlantiklachs (*Salmo salar*), Regenbogenforelle (*Oncorhynchus mykiss*), Meerforelle (*Salmo trutta*) und Wolfsbarsch (*Dicentrarchus labrax*).

5. Verwendung nach einem der Ansprüche 2 bis 4, wobei die Zusammensetzung den Fisch vor Befall durch eine parasitäre Crustaceenspezies schützt.

6. Verwendung nach Anspruch 5, wobei die Crustaceenspezies zu einer Spezies der Meerläuse gehört, einschließlich *Lepeophtheirus salmonis* und *Caligus elongatus.*

7. Verwendung nach Anspruch 6, wobei die Crustaceenspezies einer Spezies der Isopoden angehört einschließlich *Anilocra physodes.*

8. Verwendung nach einem der Ansprüche 5 bis 7, wobei die Zusammensetzung vor parasitärem Befall für einen Zeitraum von mindestens 12 Wochen nach Verabreichung der Zusammensetzung schützt.

9. Verwendung nach einem der vorhergehenden Ansprüche, wobei die Zusammensetzung für das gleichzeitige Bekämpfen von parasitärem Befall und Infektionskrankheiten bei Fischen ist, und die Zusammensetzung weiter eine immunologisch wirksame, antigene Substanz umfasst, die dem Fisch nach Verabreichung Schutz vor mindestens einer Virus- oder Bakterienspezies vermittelt.

10. Verwendung nach einem der Ansprüche 2 bis 9, wobei die Zusammensetzung in Form einer Lösung, Suspension oder Emulsion von Lufenuron formuliert ist.

11. Verwendung nach einem der Ansprüche 2 bis 10, wobei die Zusammensetzung in Form einer Emulsion von Lufenuron formuliert ist.

12. Verwendung nach einem der Ansprüche 2 bis 10, wobei die Zusammensetzung in Form einer Emulsion mit Impfstoff-Komponenten formuliert ist.

## Revendications

1. Lufenuron pour utilisation dans une metode pour contrôler les infestations parasitaires chez le poisson, dans laquelle lufenuron est administrée via injection.

2. Utilisation de lufenuron dans la fabrication d'une composition pour contrôler les infestations parasitaires chez le poisson, dans laquelle ladite composition est administrée via injection.

3. Utilisation selon la revendication 2, dans laquelle le poisson auquel la composition est administrée est du poisson d'élevage.

4. Utilisation selon la revendication 3, dans laquelle le poisson est choisi dans le groupe constitué par une espèce (*Salmo salar*), la truite arc-en-ciel (*Oncorhynchus mykiss*), la truite de mer (*Salmo trutta*) et le bar commun (*Dicentrarchus labrax*).

5. Utilisation selon l'une quelconques des revendications 2 à 4, dans laquel la compostion protège le poisson contre une infestation avec une espèce de crustacée parasitaire.

6. Utilisation selon la revendication 5, dans laquel l'espèce crustacée appartient aux espèces de poux de mer incluant *Lepeophtheirus salmonis* et *Caligus elongatus.*

7. Utilisation selon la revendication 6, dans laquel l'espèce crustacée est une espèce incluant *Anilocra physodes.*

8. Utilisation selon l'une quelconques des revendications 5 à 7, dans laquelle la compostion protège contre l'infestation parasitaire pendant uen période d'au moins 12 semaines après l'administration de la composition.

9. Utilisation selon according to any of the preceeding claims, wherein said composition is for simultaneous control of parasitic infestations and infectious diseases in fish, and the composition further comprises an antigen conferring, upon administration to the fish, immunological protection against at least one viral or bacterial species.

10. Utilisation selon l'une quelconques des revendications 2 à 9, dans laquelle la composition est formulée dans une solution, suspension ou émulsion de lufenuron.

11. Utilisation selon l'une quelconques des revendications 2 à 10, dans laquelle la composition est formulée dans une émulsion de lufenuron.

12. Utilisation selon l'une quelconques des revendications 2 à 10, dans laquelle la composition est formulée dans une emulsion de lufenuron avec des composés de vaccine.
